# EUROPEAN PATENT APPLICATION

(11) **EP 4 140 594 A1**
(43) Date of publication of application: **01.03.2023**
(21) Application number: 21193707.3
(22) Date of filing: 30.08.2021
(51) Int. Cl.: B05B 7/04, B65D 83/14, B65D 83/48

(54) **AEROSOLSPRAY**

(71) Applicant: Beiersdorf AG, 20253 Hamburg (DE)
(72) Inventor: Ripke, Sabine, 22527 Hamburg (DE); Miertsch, Heike, 22459 Hamburg (DE)

(57) **Abstract**

The present invention belongs to the cosmetic field.

## Description

The invention belongs to the cosmetic field and relates to aerosol sprays with optimized spray droplet size.

Cosmetic products generally not only serve to look beautiful and attractive, but their effect makes a decisive contribution to increased self-esteem and people's wellbeing. Accordingly, a wide variety of cosmetic products are used for daily cleaning and caring of the skin.

One category of cosmetic products are deodorants and antiperspirants, which are used under the armpits to avoid malodor. The cause of malodor is the decomposition of odorless sweat by bacteria. As a product of the decomposition, fatty acid residues occur, which characterize the typically known sweat odor. Regardless of the fact that deodorants and antiperspirants are used on the same skin area and pursue the same objective, they differ in their technical effect.

Antiperspirants contain antiperspirant active ingredients that are used to hinder and reduce the flow of sweat. For example, if aluminum-containing antiperspirant active ingredients are applied to the skin the sweat glands become clogged resulting in a reduced flow of sweat.

Deodorants, on the other hand, are characterized by the fact that they do not prevent the flow of sweat. Instead, by definition, deodorants are products that contain special antimicrobial agents that reduce the growth of bacteria such as Corynebacterium jeikeium or Staphylococcus epidermidis. As a result, the decomposition of odorless sweat is reduced, so that less malodor occurs.

Conventionally, antiperspirants and deodorants are applied to the skin either as liquid using an application roller or as spray. Both propellant-operated aerosol sprays and pump sprays operated by a hand pump may be used as sprays. Depending on the form of application, the cosmetic preparations vary significantly in their composition.

The present invention relates to cosmetic aerosol sprays for use in the armpit. In contrast to conventional aerosol sprays which are operated with liquid propellants, the present invention addresses pressurized aerosol sprays which use compressed gases like air and/or nitrogen as propellant. As a result, the use of liquid propellants such as propane or butane, which can be harmful to the environment, is avoided. Furthermore, the compressed gases are not flammable, so that accidents can be avoided.

Although some prior art documents suggest that conventional propellants such as propane or butane can easily be replaced by compressed air or nitrogen, this is not the case. The spray properties of the products differ considerably. Furthermore, a different filling pressure in the aerosol can is necessary to generate a suitable spray.

Regardless of the propellant, however, consumer requirements for aerosol sprays for use as deodorants remain the same. The consumer expects the deodorant preparation contained in the aerosol spray to be effectively transferred to the skin by means of the spray device.

One of the typical parameters characterizing the spray performance is the particle/droplet size distribution. Thereby, it is crucial that the droplet size of the aerosol particles does not become too small, since otherwise the aerosol particles could be inhaled particularly easily. This has been described in Rothe, H., Fautz, R., Gerber, E., Neumann, L., Rettinger, K., Schuh, W., Gronewold, C.; Special aspects of cosmetic spray safety evaluations: Principles on inhalation risk assessment (2011) Toxicology Letters, 205 (2), pp. 97-104.

On the other hand, overly large droplets are perceived as wet on skin leading to an unpleasant feeling for the consumer.

The documents US5082652A and US 20040144863A1 describe effects on the particle/droplet size distribution.

Several parameters influence droplet size distribution of sprays. Besides the setup of the valve and the nozzle the properties of the fluid like surface tension, volatility and viscosity are important. The lower the surface tension the easier the droplets can break when being atomized.

Regarding the cosmetic composition to be sprayed the person skilled in art would expect that the higher the surface tension the larger the sprayed droplets will become. Thus, by mixing water with ethanol the droplet size would expect to be increased with increasing water content.

Ultimately, there is a particular need for measures such as how the droplet sizes of aerosol sprays, which are operated with nitrogen as a propellant, can be adjusted such that the fraction of small droplets is reduced or shifted towards larger droplets while maintaining a reasonable spray which leaves a pleasant skin feeling in the armpit. Thereby, the risk of inhalation shall be reduced.

Surprisingly, it has now been found that the present invention addresses the points described above.

The present invention is a handheld spray device comprising
a) a body comprising a reservoir to house a total fill of materials;
b) an actuator comprising an actuator exit orifice;
c) a valve assembly in fluid communication with the actuator exit orifice and the reservoir;
d) a propellant stored in the reservoir, whereby the propellant has a concentration from 30% to 70% by volume of the total fill of materials stored within the reservoir and the propellant is nitrogen and/or compressed air; and
e) a composition, preferably a cosmetic composition, stored in the reservoir, whereby the composition comprises
   a. ethanol, and
   b. water,
   wherein the ratio by weight between ethanol and water is in the range from 5:1 to 1:4.

A further aspect of the invention is the use of the handheld spray device to spray the cosmetic composition to the human body, whereby the spray is released by actuating the actuator.

All the weight percentages (% by weight) given below relate, unless otherwise stated, to the total weight of the composition. If ratios of certain components are disclosed in the following description, these ratios refer, unless otherwise stated, to weight ratios of the components.

Unless otherwise stated, all tests and measurements were performed under "normal conditions". The term "normal conditions" refers to 20°C, 1013 hPa and a relative humidity of 50%.

In the following description the terms "according to the invention", "preferred according to the invention" and so on are always directed to the use according to the invention and to the method according to the invention.

For the purposes of the present disclosure, the term "free from" means that the proportion of the respective substance is less than 0.05% by weight. This ensures that entrainments or impurities with these substances are not included as "free from" according to the invention.

The term liquid in liquid fragrance material means that the fragrance material is liquid under normal conditions.

The term cosmetic composition is understood as a composition which is used for cosmetic purposes, whereby only ingredients are contained which are listed in the International Cosmetic Ingredient Dictionary and Handbook, Thirteenth Edition 2010, (ISBN 1-882621-47-6).

The term "skin" refers solely to the human skin.

Term "particle/droplets" refers to both, liquid and solid parts sprayed. The term "particle size distribution" includes spray populations containing liquid ingredients only.

Emulsifiers are understood to be all substances which are listed in the International Cosmetic Ingredient Dictionary and Handbook, Thirteenth Edition 2010, (ISBN 1-882621-47-6) under the name "emulsifying agent". Surfactants are understood to be all substances which are listed in the International Cosmetic Ingredient Dictionary and Handbook, Thirteenth Edition 2010, (ISBN 1-882621-47-6) under the name "surfactant".

In general spray devices comprising a body comprising a reservoir to house a total fill of materials; an actuator comprising an actuator exit orifice and a valve assembly in fluid communication with the actuator exit orifice and the reservoir or available on the market. For example, these can be purchased from Lindal, Coster und Salvalco.

### Actuator

The handheld spray device comprises an actuator which is preferably mounted on the valve assembly and which preferably incorporates a spray outlet region located at the actuator exit orifice, said actuator assembly further preferably incorporating a discharge conduit providing a communication between the stem flow conduit and the spray outlet region. The stem outlet flow conduit may be of circular-section as may be the discharge conduit. Preferably the flow and discharge conduits are of identical diameter, ideally in the range 0.5 mm to 1.5 mm. The flow and discharge conduit may each have a length from 3 to 50 times their diameter. The discharge conduit may, throughout its length, be collinear with the flow conduit. Alternatively, the discharge
conduit may be formed in two sections, namely a first section collinear with the flow conduit and a second section angled (e.g. perpendicular thereto).

### Valve assembly

According to the present invention the valve assembly is preferably configured such that a portion of the propellant from the reservoir is bled into the liquid composition being supplied to the actuator. In this way bubble-laden ("bubbly") two phase flow is created which can be atomized at the actuator exit orifice to produce the spray.

The person skilled in art is aware of several approaches to create a bubble-laden flow in the valve assembly. In the following preferred valve assemblies will be described.

### 1. Embodiment of the valve assembly

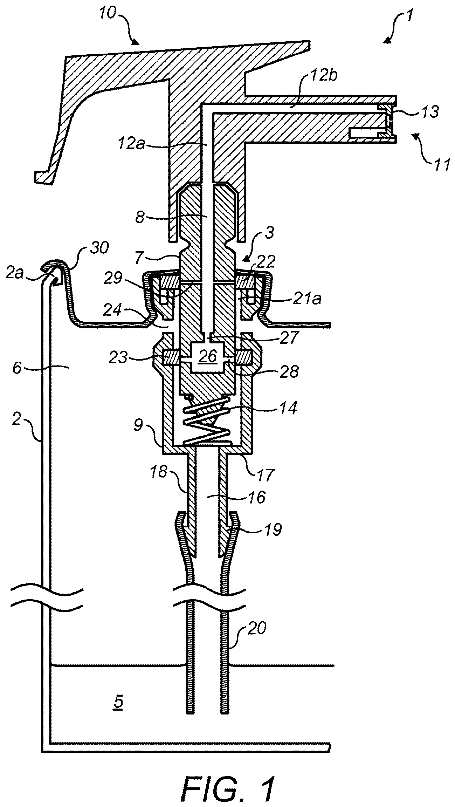

The valve assembly of the first embodiment is illustrated in Figure 1. The handheld spray device 1 is presented in the normal "closed" position.

The handheld spray device 1 comprises a reservoir 2 on the top of which is mounted an actuator 10 comprising an actuator exit orifice, which allows a spray outlet region 11 to be formed. Provided within the reservoir 2 is a composition 5 to be dispensed from the device by a pressurized gas such as nitrogen and/or air, situated in a head space 6 of the reservoir 2. The gas in the head space 6 may, for example, be at an initial pressure of 9 to 20 bar depending upon the type of container in use. The initial pressure may, for example, be 9 or 12 bar. There are however higher pressure "standard" cans now available (but as yet little used), for which the initial pressure is for example 18 bar or higher. Such cans can also be used in the present invention. Higher initial can pressure is good because there is more mass of gas available to help atomization and higher nozzle velocities which also helps atomization and also the proportionate loss in can pressure as the can empties is less. This helps maintain spray quality and flow rate better during can lifetime.

The valve assembly 3 comprises preferably a generally cylindrical, axially movable valve stem 7 having an axial bore 8 extending from the upper end of valve stem 7 part way towards the lower end thereof. At its lower (proximal) end, valve stem 7 locates within a cylindrical housing 9 positioned internally of the reservoir 2 and at its upper (distal) end is fitted with an actuator 10 in the form of a cap having a spray outlet region 11 located at the actuator exit orifice at the end of tube 12b. Provided at the actuator exit orifice is a conventional MBU (Mechanical Break-Up Unit) insert 13. The valve assembly 3 is secured to the top of the reservoir 2
by means of a metallic top cap 30 which is crimped at a central portion to the upper end of the valve housing 9 and crimped at an outer periphery to the upper rim 2a of the reservoir. An outer gasket (not shown) would typically be secured in place between the upper rim 2a and the outer periphery of the top cap 30 to ensure a hermetic seal.

The handheld spray device 1 is operated by pressing down on the actuator 10 to cause downward movement of valve stem 7 to an "open" position with resultant discharge of a spray from spray outlet region 11. As shown in the drawings, valve stem 7 is biased upwardly of the reservoir 2 by means of a coil spring 14. Lower end of coil spring 14 locates around an aperture 16 in lower wall 17 of the housing 9. Depending from wall 17 is a tubular spigot 18 having a lower enlarged end 19 to which is fitted a dip tube 20 which extends to the base of the reservoir 2. It will be appreciated from the drawing that the lower region of reservoir 2 is in communication with the interior of the housing 9 via the dip tube 20, spigot 18 and aperture 16 (which provides a liquid inlet for housing 9).

The valve assembly includes a pair of sealing gaskets: a first 23 dedicated to sealing liquid inlets 28 to the stem; and a second 21 dedicated to sealing gas inlets 29 to the stem. The annular gaskets 22 and 23 are formed of rubber or other elastomeric material and are dimensioned to seal against the outer surface of valve stem 7. Formed in the wall of the housing 9 between the two gaskets 22 and 23 are a plurality of ports 24 which provide for communication between the pressurized gas in the head space 6 and an annular clearance 21 a.

The liquid feed passageways 28 and gas bleed inlet passageways 29 are axially spaced from each other by a distance such that, in the "rest" condition ("closed" position) of the aerosol as shown in figure 1, the passageways 29 are sealed by upper gasket 22 and passageways 28 are sealed by lower gasket 23. The cross-sections of the passageways 28 and 29 together with the axial spacing between these passageways and the dimensions of the upper and lower gaskets 22 and 23 are such that on depression of the valve stem 7 to the open position the gas bleed inlet passageways 29 are opened simultaneously with (or more preferably just before) the liquid feed passageways 28, thereby causing the generation of bubble laden flow in the outlet conduit 8 for supply to the spray outlet region 11 the tube 12a and 12b and the actuator exit orifice located at the end of tube 12b for discharge therefrom in the form of a fine aerosol.

### 2. Embodiment of the valve assembly

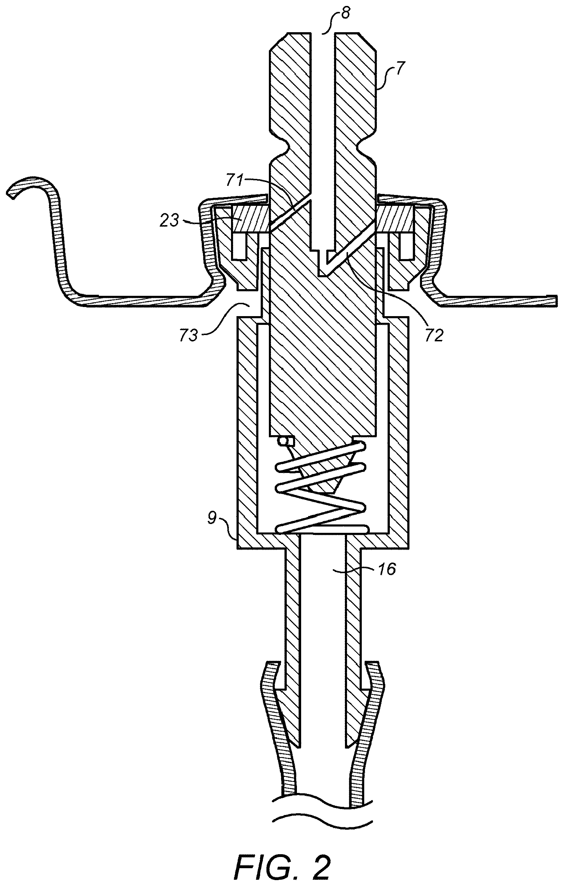

Another preferred embodiment is presented in Figure 2. The valve assembly presented in Figure 2 shows that a single gasket 23 is used to seal both the liquid inlet 72 to the stem and the gas inlet 71 to the stem. On movement of the valve stem 7 from the closed position to the open position, the stem inlets 71, 72 are moved proximally of the gasket 23 and are therefore brought into fluid communication with, respectively, a gas inlet 73 in the housing 9, and a liquid inlet 16 in the housing, thereby causing the generation of bubble laden flow in the outlet conduit 8.

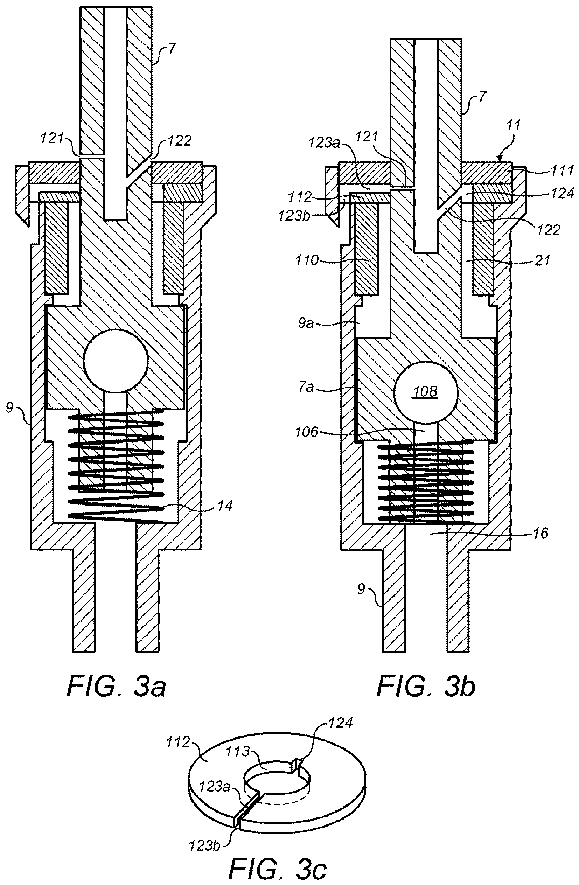

Further preferred embodiments of the invention are presented in Figure 3a, 3b, and 3c. Fig 3a shows the valve stem 7 of a single gasket valve assembly in a closed position, in which the valve stem 7 is extended out of the housing 9, under the action of the spring 14, so that the gas bleed inlet(s) 121 and the liquid inlets(s) 122 are each on the opposite (distal) side of the seal 23 to the gasket 112, or are at least blocked by the seal 23. The thin gasket 112 is shown in greater detail in Fig 3c and comprises a disc having a central aperture 113 that is sized to be a close fit about the valve stem 7. A radial groove 123a extends in one side of the disc from the central aperture to an edge of the disc, where the groove connects with an axial notch 123b that extends through the edge of the disc. The groove 123a and notch 123b together comprise a gas inlet port that forms a gas flow path from the headspace 6 to the gas bleed inlet 121 when the valve stem is depressed, as in Fig 3b. A notch 124 extends through the disc 112 at a point at the edge of the aperture 113 diametrically opposite to the groove 123a. When the valve stem is depressed, the notch 124 forms a liquid flow path between the annular clearance 21 and the liquid feed inlet 122. The annular clearance 21 is in fluid communication with the liquid inlet 16 in the housing via an axial channel 106 through the lower portion of the valve stem 7 and a transverse opening 108 located at the upper end of the channel 106. An advantage of a single gasket arrangement is that it employs fewer parts and thus reduces material, manufacturing and assembly costs in comparison to double gasket arrangements.

### 3. Embodiment of the valve assembly

Another preferred valve assembly comprises a housing with internal walls defining a valve chamber, the chamber having a liquid inlet for fluid communication with liquid in the reservoir of the handheld spray device, and a gas inlet for fluid communication with gas in the reservoir; and a valve stem having proximal and distal ends, the proximal end received in the valve chamber and the distal end projecting through a sealed opening in the valve chamber, the valve stem including an outlet flow conduit with an outlet aperture at the distal end and, more proximally, at least one first stem inlet for liquid and at least one second stem inlet for gas; wherein the housing includes a lip projecting inwardly from the internal walls to form a seal around a perimeter of the valve stem along at least a portion of the valve stem, wherein the valve chamber liquid inlet is proximal of the lip and the valve chamber gas inlet is distal of the lip; wherein the valve stem is moveable between:
a closed position in which the at least one first stem inlet is distal of the lip and the at least one second stem inlet is distal of the sealed opening in the valve chamber, such that the at least one first stem inlet is not in fluid communication with the valve chamber liquid inlet and such that the at least one second stem inlet is not in fluid communication with the valve chamber gas inlet; and an open position in which the at least one first stem inlet is proximal of the lip so as to be in fluid communication with the valve chamber liquid inlet, and the at least one second stem inlet is proximal of the sealed opening in the valve chamber and at least partially distal of the lip so as to be in fluid communication with the valve chamber gas inlet, whereby a bubble laden flow is created in the flow conduit.

This valve assembly allows that the liquid flow path is kept separate from the gas flow path (until the valve is in the open position, when the liquid and gas mix in the outlet flow conduit) by virtue of the sealing interface between the lip and the valve stem, rather than by a sealing gasket. The liquid thus never comes into contact with the gasket, and accordingly swelling of the gasket due to such contact is avoided.

The at least one second stem inlet for gas is preferably downstream of said at least one first stem inlet for liquid.

The valve stem is typically biased towards the closed position.

The valve assembly may further comprise a limit stop to prevent movement of the valve stem distally beyond the closed position. The limit stop may comprise a shoulder projecting radially from the valve stem towards the proximal end thereof for abutment against said lip. The shoulder may include a channel which, when the valve stem is in the open position, allows fluid to flow from the valve chamber liquid inlet to the at least one first stem inlet, but which when the valve stem is in the closed position is closed off by the abutment against the lip, preventing the flow of liquid through the channel. The channel may comprise at least one radially extending conduit in fluid communication at one end thereof, in the centre of the valve stem, with a bore from the distal end of the valve stem, and at the other end thereof with a groove in the outer surface of the shoulder running parallel to the bore and to the outlet conduit.

At least the portion of the valve stem about which the lip forms a seal preferably has a constant cross-section. Typically, the valve stem has a circular cross-section.

The housing may comprise a cup portion and a cap portion. The valve chamber liquid inlet may be formed through the cup portion, and the valve chamber gas inlet may be formed through the cap portion.

The valve chamber gas inlet may comprise a plurality of radial grooves defined between corresponding radial ribs on an upper surface of the housing, in conjunction with a conduit through the housing to the outer surface thereof, for communication with the headspace of a container to which the spray device is fitted.

The sealed opening is typically sealed by a gasket, which is preferably a planar, annular gasket. Where the valve chamber gas inlet comprises a plurality of radial grooves defined between corresponding radial ribs on an upper surface of the housing, the gasket preferably also defines an upper bound of the radial grooves in the housing. In other embodiments, it is necessary to provide a separate part to support the gasket within the housing, such as the support ring 110 of Figs 3a and 3b. That is not necessary with the inventive arrangement, in which the upper surface of the housing has a dual purpose of supporting the gasket and defining (part of) the gas flow path.

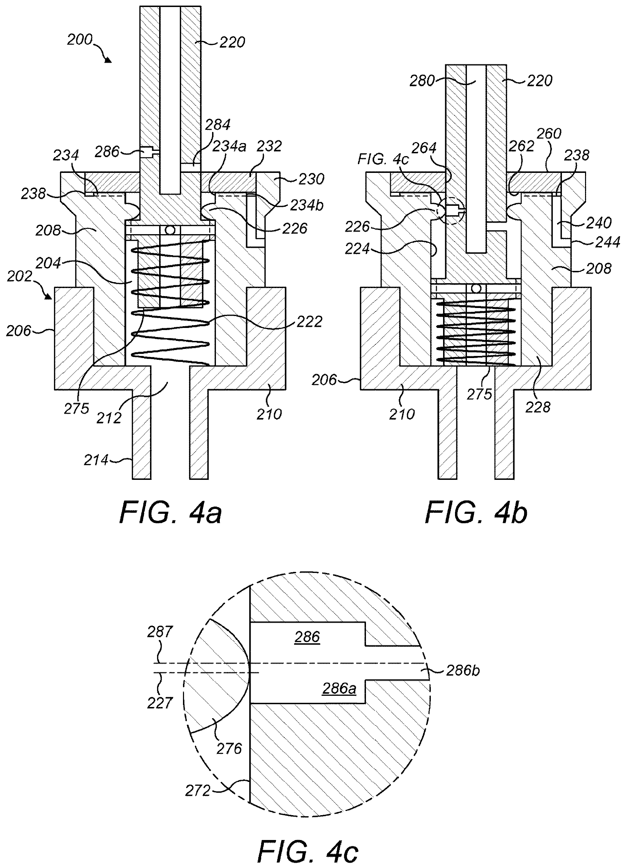

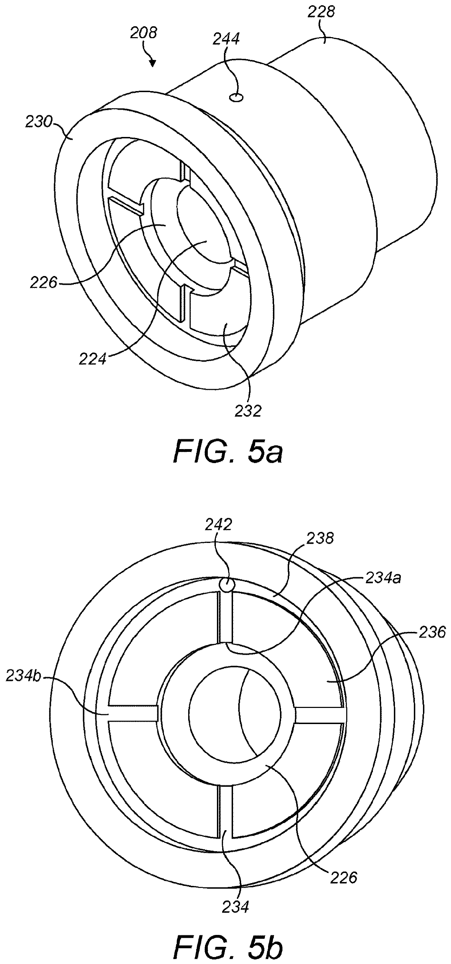

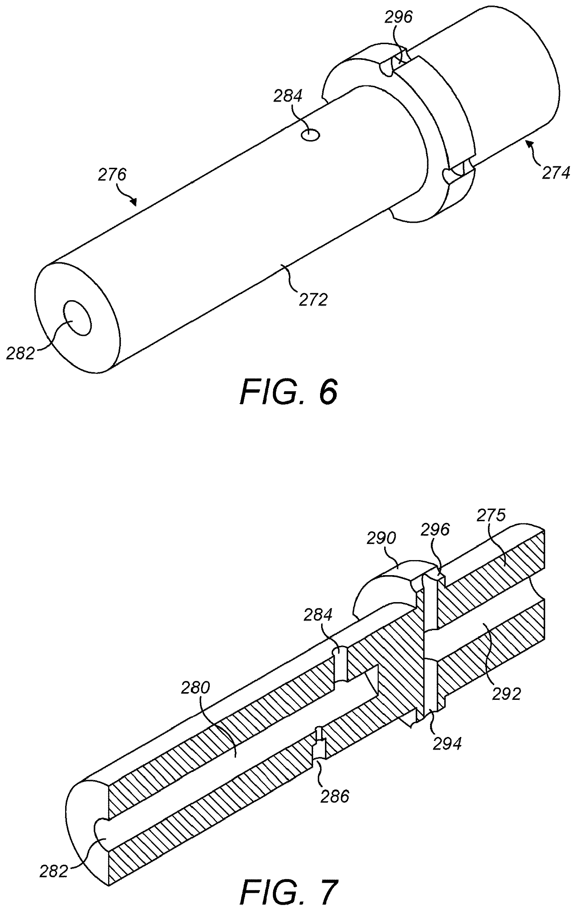

A valve assembly 200 according to the third embodiment is illustrated in the accompanying Figures 4a to 7. Used in the handheld spray device according to the invention.

The valve assembly 200 comprises a housing 202 with internal walls defining a valve chamber 204, and a valve stem 220. The housing 202 is formed of two portions: a lower, cup portion 206; and an upper, cap portion 208. The valve assembly 200 would be crimped in place at the top of a reservoir, with a distal portion of the valve stem 220 projecting from the top of the reservoir for connection to the actuator.

The cup portion 206 has a lower wall 210 with an aperture 212 therethrough. A tubular spigot 214 depends from the lower wall 210. A dip tube (not shown) would be connected to the tubular spigot 214, typically by means of an enlarged lower end as described by Figure 1, the dip tube extending to the base of the reservoir to which the valve assembly 200 is fitted. It will be appreciated that the lower region of a reservoir to which the valve assembly 200 is fitted is in communication with the valve chamber 204 via the dip tube, spigot 214 and aperture 212 (which provides a liquid inlet for the valve chamber).

The cap portion 208 comprises a generally cylindrical inner wall 224 from which a lip 226 projects inwardly at the upper end thereof. The lower end 228 of the cap portion has a narrower outer diameter so as to fit with an interference fit inside the cup portion 206. At the upper end of the cap portion 208, an annular rim 230, together with an upper surface 232, defines a shelf within which an annular sealing gasket 260 sits.

A plurality of radial grooves 234 are defined between corresponding radial ribs 236 on the upper surface 232. Inner ends 234a of the grooves 234 open into the upper end of the valve chamber, above the lip 226. Outer ends 234b of the grooves 234 open into a circumferential groove 238, which circumscribes the upper surface 232 just inside the rim 230. The lower and side surfaces of the respective grooves 234, 238 are formed by the cup portion itself, whereas the upper surfaces thereof are formed by the lower surface 262 of the gasket 260.

A conduit 240 is formed through the cap portion 208, with an upper end opening into the circumferential groove 238 via a hole 242, and with a lower end exiting the side of the cup portion via a hole 244 in the outer surface thereof. It will be appreciated that the head space of a container to which the valve assembly 200 is fitted is in communication with the valve chamber 204 via the conduit 240, circumferential groove 238 and radial grooves 234 (which together provide a gas inlet for the valve chamber).

The valve stem 220 is generally cylindrical, having an outer surface 272 with a diameter equal to the inner diameter of the lip 226 such that the lip 226 forms a seal around the perimeter of the valve stem. A proximal end 274 of the valve stem is received in the valve chamber 204 and a distal end 276 projects through the centre 264 of the annular sealing gasket 260, which is dimensioned to seal against the outer surface 272 of the valve stem 220. The lower surface 262 of the gasket 260 defines the top of the valve chamber 204.

The valve stem 220 includes an outlet flow conduit 280 with an outlet aperture 282 at the distal end 276 and, more proximally, at least one first stem inlet 284 for liquid and at least one second stem inlet 286 for gas. As illustrated, there is a single stem inlet 284 for liquid and a single stem inlet 286 for gas, and they are positioned roughly in the middle of the valve stem, with the gas inlet 286 being slightly distal of the liquid inlet 284. It will be understood that alternative arrangements are envisaged. For example, there could be multiple liquid inlets 284 and/or multiple gas inlets 286; the inlets 284, 286 could be located more proximally or more distally than shown; and the axial separation between the respective liquid and gas inlets could be greater than shown.

Towards the proximal end 274 of the valve stem 220, an enlarged shoulder portion 290 projects radially from the cylindrical valve stem 220. The diameter of the shoulder 290 is substantially equal to that of the valve chamber 204. A bore 292 runs centrally from the proximal end face 275 valve stem 220 to the shoulder portion 290. Four conduits 294 extend radially within the shoulder portion 290 from the center, where they open into the bore 292, to the outside. At the outer ends, the radial conduits 294 open into respective axial grooves 296 in the outer surface of the shoulder 290 that run parallel to the bore 292 and to the outlet conduit 280.

As shown in the drawings, the valve stem 220 is biased upwardly of the valve assembly (and thus of the handheld spray device) by means of a coil spring 222. Lower end of coil spring 222 locates around the aperture 212 of the cup portion 206 of the housing 202. In the closed valve position, as shown in Figure 4a, the shoulder 290 abuts against the lip 226 under the force of the spring 222, and the flow channel defined by the bore 292, radial conduits 294 and axial grooves 296 is blocked by virtue of the tops of the axial grooves 296 abutting against the underside of the lip 226. Furthermore, the liquid inlet 284 is more distal than the sealing gasket 260. Accordingly, there is no fluid communication between the valve chamber liquid inlet 212 and the outlet conduit 280. There is also no fluid communication between the valve chamber gas inlet 234a and the outlet conduit 280, because the gas inlet 286 is also more distal than the sealing gasket 260, which hermetically seals against the outer surface 272 of the valve stem.

The abutment of the shoulder 290 against the lip 226 acts as an upper limit stop, preventing the valve stem 220 from being urged further out of the valve housing 202.

When the valve stem is moved to the open position, as shown in Figure 4b, the stem liquid inlet 284 is moved below (i.e. proximal of) the lip 226 so as to be in fluid communication with the valve chamber liquid inlet 212 via the flow channel defined by the bore 292, radial conduits 294 and axial grooves 296 through the stem shoulder portion 290. Also, the stem gas inlet 286 is moved below (i.e. proximal of) the sealing gasket 260 to a position at the upper end of the valve chamber 204 in fluid communication with the valve chamber gas inlet 234a. At least a part of the stem gas inlet 286 must be open to the upper portion of the valve chamber 204 (i.e. the portion above the lip 226). Abutment of the bottom face 275 of the valve stem 220 against the lower wall 210 of the cup portion 206 defines a lower limit stop.

To operate the handheld spray device of the invention, an actuator 10 is depressed so that the valve stem 220 moves downwardly against the bias of spring 222 from the closed position to the open position. As a result, the liquid and gas stem inlets284, 286 are displaced past the gasket 260 and brought into respective fluid communication with the composition 5 from the reservoir 2 and compressed gas from the head space 6.

Compressed gas can now flow into the outlet conduit 280 by passage through the hole 244 in the outer surface of the cap portion 208, the conduit 240, the hole 242, the circumferential groove 238 and radial grooves 234, and through the stem gas inlet 286.

The composition 5 can now flow into the upper portion of the valve chamber 204 by passage upwardly along the dip tube 20, through the inlet 212, the bore 292, the radial conduits 294 and the axial grooves 296. The composition 5 introduced into the upper portion of the valve chamber 204 passes via stem liquid inlet 284 into flow conduit 280 where it is mixed with the compressed gas bled through the stem gas inlet 286. A bubble laden flow of homogeneous bubbles with similar diameters and without significant coalescence or stratification is formed in the outlet flow conduit 280.

That bubbly flow can then flow, preferably undisturbed, through the actuator 10, such as one of the type disclosed in Figure 1, to a spray outlet region 11. This actuator 10 (which may be of the type available under the name "Kosmos" from Precision Valve (UK) Ltd) is moulded so as to locate on the top of valve stem 7, 220 and has an internal L-shaped conduit formed as a first section 12a collinear with the outlet bore 8, 280 of valve stem 7, 220 and a second section 12b that extends at right angles to section 12a and leads to spray outlet region 11. Other different actuators could be used instead; a number of different exemplary styles are disclosed in WO 2011/061531 and WO 2011/128607. The substantially disturbance-free flow of the bubble laden flow can be achieved by configuring the outlet flow conduit 280 and the flow conduit through the actuator such that there is an absence of any flow disturbances, whereby the bubble laden flow is delivered to the spray outlet region in substantially the form in which it was created.

The bubble laden flow should be at a velocity that gives a sufficiently short residence time of the flow in the outlet flow conduit 280 and the flow conduit through the actuator such that bubble coalescence or stratification does not occur. Typically, the flow rate should be in the range 0.5 to 5 m/s.

The bubble laden flow should be at between 1 bar and 20 bar pressure, and in a preferred embodiment for a consumer aerosol can, between 4 bar and 12 bar (said pressure reducing during evacuation of the can).

The ratio of volume of gas/volume of liquid contained in the bubble laden flow in the outlet flow conduit 280 should be between 0.2 and 3.0 at the pressure prevailing in this conduit and more preferably between 0.3 and 1.3.

Preferably, the conduits and outlet region (including any MBUs 13 that might be required) of the actuator 10 can be selected so as to be ideally suited to the flow and aerosolisation of whichever composition is to be dispensed therefrom.

Preferably, as shown in Figure 4c, the stem gas inlet 286 is moved to a position in which it is marginally offset distally from the lip 226 - i.e. a central axis 287 of the stem gas inlet 286 is just above the centreline 227 of the lip 226. This allows not only gas from the valve chamber gas inlet 234a to enter the stem gas inlet 286, but also a small amount of liquid from the valve chamber liquid inlet 212 too.

Preferably, the stem gas inlet 286 is stepped, having an outer portion 286a (opening to the stem surface 272) with a larger diameter than an inner portion 286b (opening to the outlet conduit 280). Alternatively, the stem gas inlet 286 may have a conical cross-section, tapering from a larger outer portion to a smaller inner portion. The advantage of such gas inlet profiles is to assist in manufacture: when moulding the valve stem, pins are typically inserted into the mould to provide for the respective gas and liquid inlets. By having a tapered or stepped profile to the gas inlet, the corresponding pin can have a matching profile, thereby being thicker and stronger at its root than would be the case with a constant diameter pin (matching the narrowest diameter required for the gas inlet). However, a constant diameter gas inlet 286 could be used instead.

In the construction of the valve assembly 200, it should be ensured that the total cross-sectional area of the gas bleed passageways 240, 238, 234, 286 should not be so large that excessive gas is bled into the outlet conduit 280 such that the container 2 is depleted of pressurized gaseous propellant before all of the liquid 5 in the container has been discharged. Typically, the total cross-sectional area of the gas bleed inlet passageways should be equivalent to that of a singular, circular section inlet with a diameter of 0.15-0.8 mm.

Preferred dimensions for the construction of the valve assembly 200 to ensure production of a bubble laden flow of homogeneous bubbles with similar diameters and without coalescence or stratification are shown in the following table:

| **Item** | **Reference Numeral** | **Diameter (mm)** | **Length (mm)** |
|---|---|---|---|
| **Stem** | | | |
| Portion of valve stem above shoulder | 272 | 3.2 | 11.4 |
| Portion of valve stem below shoulder | 274 | 3.5 | 3.65 |
| Stem shoulder portion | 290 | 4.7 | 1.0 |
| Outlet conduit in valve stem | 280 | 1.0 | 10 |
| Stem liquid inlet | 284 | 0.5 | 1.1 |
| Stem gas inlet | 286 | 0.2 | 1.1 |
| Outer portion of stem gas inlet | 286a | 0.5 | 0.7 |
| Inner portion of stem gas inlet | 286b | 0.2 | 0.4 |
| Distance of stem gas inlet from distal end of stem | | | 7.8 |
| Distance of stem liquid inlet from distal end of stem | | | 8.6 |
| Stem bore | 292 | 1.0 | 4.4 |
| Radial conduit | 294 | 0.5 | 1.6 |
| Axial groove | 296 | 0.5 (0.25 radius) | 1.0 |
| Housing | | | |
| Cup portion outer diameter | 206 | 12 | 5.4 |
| Cup portion inner diameter | | 8.0 | 4.2 |
| Spigot | 214 | 4.0 | 4.8 |
| Aperture | 212 | 2.0 | 6.0 |
| Cap portion lower end | 228 | 8.0 | 4.2 |
| Inner wall | 224 | 4.8 | |
| Lip | 226 | 3.2 | 0.91 |
| Rim | 230 | 11.5 | 1.1 |
| Circumferential groove | 238 | 9.1 | 0.5 (width); 0.2 (height) |
| Gas hole | 242 | 0.5 | |
| Gas Hole | 244 | 0.5 | |
| Conduit | 240 | 0.5 | |
| Radial groove | 234 | 0.5 | |
| Offset: stem gas inlet to li (in open position) | 227/287 | | 0.06 |

With the dimensions as indicated above, the valve assembly 200 is particularly suitable for consumer aerosol products such as polishes, insecticides, deodorants, hairspray and air fresheners.

It will be appreciated that the specific dimensions and arrangement of the various constituent parts of the respective gas and liquid flow paths are by way of example only and that alternative arrangements are envisaged. What is key is for the valve chamber gas inlet 234a to be distal of the lip 226 and for the valve chamber liquid inlet 212 to be proximal of the lip 226, whilst the stem gas and liquid inlets are positioned such that the stem liquid inlet is brought into fluid communication with the valve chamber liquid inlet and the stem gas inlet is brought into fluid communication with the valve chamber gas inlet on actuating the valve to the open position.

In particular, the arrangement of the flow passage 292, 294, 296 through and past the stem shoulder portion 290 could be omitted, so long as the stem liquid inlet is only brought into fluid communication with the valve chamber liquid inlet in the open position; the flow path being blocked by virtue of the lip 226 when in the closed position.

Also, whereas the valve assembly is described as having four radial conduits 294 and associated axial grooves 296, there may be fewer or more. Likewise, four radial grooves 234 are illustrated, but there may more or fewer.

Furthermore, although described as generally cylindrical, the stem 220 may take other generally prismatic profiles (such as square), with appropriate adaptation of mating parts such as the gasket 260 and the lip 226 and the inner walls 224 of the cap portion 208. Similarly, the shape of the outer surface of the housing 202 does not have to be generally round in cross-section.

For a given exit orifice size the dependency of gas and liquid flow rates on gas and liquid inlet diameters is complex; for example it is proposed that reducing the liquid inlet diameter produces a lowering of pressure inside the conduit which increases the inflow of gas into the conduit. However, this increased gas inflow can increase the blockage of the bubbly flow at the swirl inlets and exit orifice of an MBU, which produces a lowering of the liquid inflow rate from the value expected.

To minimize the droplet sizes, it is necessary to maximize the gas/liquid volume ratio however smaller exit orifices and higher canister pressures also reduce drop size. The ratio of volume of gas/volume of liquid contained in the bubble laden flow in the flow conduit should typically be between 0.2 and 3.0 at the pressure prevailing in this conduit and more preferably between 0.3 and 1.3, although ratios as high as 9.0 can still produce satisfactory results.

### Method of assembly

In the valve assemblies described in Figures 1 and 2, the stem 7 is typically inserted into the housing 9 from above (after dropping in the spring 14, or having already attached the spring to the bottom of the valve stem), and the spray device can then be crimped together with the top cap 30, securing the sealing gasket(s) in place and securing the assembly to a reservoir 2 . By virtue of the lip 226, and the shoulder 290 of the present invention, it would not be possible to insert the valve stem 220 into the housing 202 from above. Accordingly, a modified assembly process is carried out.

In essence, the further assembly is initially carried out upside-down. Reference to upper and lower portions, etc., should be taken as references to those portions in their usual orientation in use (i.e. an upper portion is closer to the top of a valve assembly and to the outlet spray region of a container to which it is attached than a lower portion).

Thus, to assemble a valve assembly 200 according to the invention, a gasket 260 is placed into the central portion of an inverted top cap 30, and an inverted valve cap portion 208 is placed on top, so that the gasket 260 is held in place between the top cap 30 and the shelf on the 'upper' surface 232. A valve stem 220 is inserted, distal end 276 first, through the cap portion 208 in the direction from the narrower 'lower' end 228 towards the upper surface 232. The distal end 276 passes through lip 226 with an interference fit until the shoulder 290 abuts against the lip 226. The spring 222 can then be slid over the 'lower' proximal end 274 of the valve stem. Alternatively, the spring 222 could be inserted together with the stem 220. The cup portion 206 can then be snap-fitted onto the cap portion 208.

The assembled top cap 30, housing 202 and stem 220 can then be inverted (to the upright orientation) for crimping of the central portion of the top cap 30, to secure the cap portion 208 thereto, ensuring that the hole 244 is not obstructed by the crimped top cap 30 to ensure that the gas flow passageway is viable. A dip tube 20 can then be secured to the spigot 214 at the bottom of the cup portion 206.

Alternative orders of the assembly steps can readily be envisaged, such as assembling the cup and cap portions 206, 208 of the valve housing together (after the insertion of the stem 207 and spring 222 into the cap portion 208) prior to placement onto the top cap 30 with gasket 260, or placing the gasket 260 on to the top of the assembled cup and cap portions after having been inverted to the upright orientation, then placing the top cap 30 over the gasket and valve housing combination prior to crimping. Moreover, the crimping step and the fitting of the dip tube could instead take place with the assembly in an inverted orientation.

### Propellant

The handheld spray device according to the invention comprises a propellant stored in reservoir, whereby the propellant has a concentration from 30% to 70% by volume of the total fill of materials stored within the reservoir and propellant comprises nitrogen and/or compressed air.

According to the invention it is preferred if the pressure in the reservoir is in the range from 12 bar to 3 bar, preferably 10 bar to 4 bar and most preferred from 9 to 6 bar for the case the spray device is fully filled and not used yet.

### Composition

According to the invention the composition situated in the reservoir contains ethanol and water, wherein the ratio by weight between ethanol and water is in the range from 5:1 to 1:4, preferably from 3:1 to 1:3 and most preferably from 2:1 to 1:1.

Further it is preferred if the total quantity of water in the composition is in the range from 18 to 62% by weight, more preferably from 25 to 55 % by weight and most preferably 30 to 50% by weight, calculated to the total weight of the composition.

Further it is preferred if the total quantity of ethanol in the composition is in the range from 35 to 85% by weight, more preferably from 45 to 75% by weight and most preferably 50 to 65% by weight, calculated to the total weight of the composition.

It was further surprisingly noted that further ingredients may further balanced the spray by minimizing both, very small droplets and large droplets to keep a pleasant skin feeling in the armpit and reducing the risk of inhalation.

It is preferred according to the invention if the composition comprises at least one polyglyceryl ester. According to the invention, polyglyceryl ester are understood to mean an ester which is formed from polyglycerol (for example polyglycerol-10) and at least one fatty acid.

Polyglycerol esters which are advantageous according to the invention are polyglyceryl-10 caprates, polyglyceryl-10 laurates, polyglyceryl-10 trilaurates, polyglyceryl-10 myristates, polyglyceryl-10 dimyristates, polyglyceryl-10 oleates, polyglyceryl-10 dioleates, polyglyceryl-10 dioleates, polyglyceryl-10-distal glycerates, polyglyceryl-10-distilled glycerates -10 diisostearates, polyglyceryl-6 caprylates, polyglyceryl-6 dicaprylates, polyglyceryl-6 oleates, polyglyceryl-6 ricinoleates, polyglyceryl-6 stearates, polyglyceryl-6 behenates, polyglyceryl-5 laurates, polyglyceryl-5 polyolglycerates, polyglyceryl-5 polyglycerates, polyglyceryl-5 Dioleates, Polyglyceryl-5 Stearate, Polyglyceryl-4 Caprate, Polyglyceryl-3 Caprate, Polyglyceryl-3 Cocoate, Polyglyceryl-3 Caprate / Caprylate / Succinate, Polyglyceryl-3 Distearate and/or Polyglyceryl-2 Caprate.

It is also advantageous if the polyglyceryl ester or esters are formed from a polyglycerol with 2 to 6 glycerol subunits and at least one fatty acid having 6 to 14 carbon atoms. It is particularly advantageous if the polyglyceryl ester or esters are formed from a polyglycerol with 2 glycerol subunits and at least one fatty acid having 8 to 12 carbon atoms.

Polyglyceryl-2 Caprate is particularly preferred.

The total proportion of the polyglyceryl ester is advantageously from 0.1 to 8.0% by weight, preferably from 0.2 to 2.0% by weight and particularly preferably from 0.4 to 1.5% by weight, based on the total weight of the composition.

It is particular preferred if Polyglyceryl-2 Caprate is contained in a total quantity from 0.1 to 8.0% by weight, preferably from 0.2 to 2.0% by weight and particularly preferably from 0.4 to 1.5% by weight, based on the total weight of the composition.

Further it is preferred if the composition comprises at least one polyol having 3 to 10 carbon atoms. Preferred polyols are propanediol, propylene glycol, butylene glycol, glycerol,1,2 propanediol, 1,2 hexanediol, 1,2 heptanediol and decylene glycol. Amon the polyols listed it is most preferred if glycerol is contained in the composition.

According to the invention it is preferred if at least one polyol having 3 to 10 carbon atoms is contained in a total quantity in the range from 0.1 to 6% by weight, more preferably 1 to 5% by weight and most preferably 2 to 4% by weight, calculated to the total weight of the composition.

According to the invention it is preferred if at least one polyol selected from the group consisting of propanediol, propylene glycol, butylene glycol, glycerol,1,2 propanediol, 1,2 hexanediol,1,2 heptanediol and decylene glycol is contained in a total quantity in the range from 0.1 to 6% by weight, more preferably 1 to 5% by weight and most preferably 2 to 4% by weight, calculated to the total weight of the composition.

According to the invention it is preferred if glycerol is contained in a total quantity in the range from 0.1 to 6% by weight, more preferably 1 to 5% by weight and most preferably 2 to 4% by weight, calculated to the total weight of the composition.

The composition of the invention may further comprise one or more natural extract. Those can be contained in quantities of 0.001 to 2 % by weight of the composition.

According to the invention it is thus preferred if the composition comprises one further liquid fragrance material. The liquid fragrance material may preferably be any natural or synthetic perfume component known to one skilled in the art of creating fragrances. Those are preferably selected from essential oils, citrus oils, absolutes, resinoids, resins, concretes, etc., and synthetic perfume components such as hydrocarbons, alcohols, aldehydes, ketones, ethers, acids, esters, acetals, ketals, nitriles, etc., including saturated and unsaturated compounds, aliphatic, carbocyclic and heterocyclic compounds. Some explicitly preferred liquid fragrance materials are geraniol, geranyl acetate, linalool, linalyl acetate, tetrahydrolinalool, citronellol, citronellyl acetate, dihydromyrcenol, dihydromyrcenyl acetate, tetrahydromyrcenol, terpineol, terpinyl acetate, nopol, nopyl acetate, 2-phenylethanol, 2-phenylethyl acetate, benzyl alcohol, benzyl acetate, benzyl salicylate, benzyl benzoate, styrallyl acetate, amyl salicylate, dimethylbenzyl carbinol, trichloromethylphenyl-carbinyl acetate, p-tert.butyl-cyclohexyl acetate, isononyl acetate, vetiveryl acetate, vetiverol, alpha-n-amylcinammic aidehyde, alpha-hexylcinammic aldehyde, 2-methyl-3-(p-tert.butylphenyl)-propanol, 2-methyl-3-(p-isopropylphenyl)-propanal, 3-(p-tert.butylphenyl)-propanal, tricyclodecenyl acetate, tricyclodecenyl propionate, 4-(4-hydroxy-4-methylpentyl)-3-cyclohexene carbaldehyde, 4-(4-methyl-3-pentenyl)-3-cyclohexene carbaldehyde, 4-acetoxy-3-pentyltetrahydropyran, methyldihydrojasmonate, 2-n-heptylcyclopentanone, 3-methyl-2-pentylcyclopentanone, n-decanal, 9-decenol-1, phenoxyethyl isobutyrate, phenyl-acetaldehyde dimethyl acetal, phenylacetaldehyde diethyl acetal, geranonitrile, citronellonitrile, cedryl acetate, 3-isocamphylcyclohexanol, cedryl methyl ether, isolongifolanone, aubepine nitrile, aubepine, heliotropine, coumarin, eugenol, vanillin, diphenyl oxide, hydroxycitronellal, ionones, methylionones, isomethylionones, irones, cis-3-hexenol and esters thereof, indane musk fragrances, tetralin musk fragrances, isochroman musk fragrances, macrocyclic ketones, macrolactone musk frangrances, ethylene brassylate and aromatic nitro-musk fragrances. Further perfume components which may be included but which are not explicitly preferred are described in Arctander, Perfume and Flavour Chemicals (Chemicals), Vol. I and II (1969) and Arctander, Perfume and Flavour Materials of Natural Origin (1960).

Preferred embodiments of the invention are characterized in that the composition contains from 0.2 to 4% by weight, more preferably from 0.3 to 3% by weight and most preferably of 0.4 to 2.5% by weight of at least one liquid fragrance material, whereby the composition comprises at least one liquid fragrance material selected from geraniol, geranyl acetate, linalool, linalyl acetate, tetrahydrolinalool, citronellol, citronellyl acetate, dihydromyrcenol, dihydromyrcenyl acetate, tetrahydromyrcenol, terpineol, terpinyl acetate, nopol, nopyl acetate, 2-phenylethanol, 2-phenylethyl acetate, benzyl alcohol, benzyl acetate, benzyl salicylate, benzyl benzoate, styrallyl acetate, amyl salicylate, dimethylbenzyl carbinol, trichloromethylphenyl-carbinyl acetate, p-tert.butyl-cyclohexyl acetate, isononyl acetate, vetiveryl acetate, vetiverol, alpha-n-amylcinammic aidehyde, alpha-hexylcinammic aldehyde, 2-methyl-3-(p-tert.butylphenyl)-propanol, 2-methyl-3-(p-isopropylphenyl)-propanal, 3-(p-tert.butylphenyl)-propanal, tricyclodecenyl acetate, tricyclodecenyl propionate, 4-(4-hydroxy-4-methylpentyl)-3-cyclohexene carbaldehyde, 4-(4-methyl-3-pentenyl)-3-cyclohexene carbaldehyde, 4-acetoxy-3-pentyltetrahydropyran, methyldihydrojasmonate, 2-n-heptylcyclopentanone, 3-methyl-2-pentylcyclopentanone, n-decanal, 9-decenol-1, phenoxyethyl isobutyrate, phenyl-acetaldehyde dimethyl acetal, phenylacetaldehyde diethyl acetal, geranonitrile, citronellonitrile, cedryl acetate, 3-isocamphylcyclohexanol, cedryl methyl ether, isolongifolanone, aubepine nitrile, aubepine, heliotropine, coumarin, eugenol, vanillin, diphenyl oxide, hydroxycitronellal, ionones, methylionones, isomethylionones, irones, cis-3-hexenol and esters thereof and ethylene brassylate.

It is most preferred if liquid fragrance materials are contained in a total quantity in the range from 0.1 to 2.0% by weight, calculated to the total weight of the composition.

In order to enable further deodorizing effects, one or more additional deodorizing active ingredients are advantageously added to the compositions according to the invention.

These deodorizing active ingredients can preferably be selected from cationic polymers, in particular polyquaternium-16, polyaminopropyl biguanide and epsilon-polylysine.

Further preferred deodorizing active ingredients may be selected from the group consisting of octenidinhydro chloride, alexidindihydro chloride, benzyl alcohol, benzalkonium chloride, cetyltrimethylammonium chloride, silver citrate, triclosan, ethylhexylglycerin, triethyl citrate, 2-butyloctanoic acid, Methylphenylbutanol, phenoxyethanol and zinc ricinoleate.

Further it is preferred if the composition is free from aluminum salts. That means that it is preferred if the content of aluminum salts is equal to 0.0% by weight, calculated to the total weight of the composition.

The cosmetic compositions according to the invention can also contain cosmetic auxiliaries and active ingredients, as they are usually used in such compositions, e g. active ingredients, preservatives, preservatives, bactericides, lipids, substances to prevent foaming, dyes and color pigments, thickeners, moisturizing and / or moisturizing substances or other common components of a cosmetic or dermatological formulation such as polyols, polymers, foam stabilizers, organic solvents or silicone derivatives provided that the additive does not impair the required properties in terms of viscosity, pH values and AT effect or is excluded.

The following example shall illustrate the invention in further manner. For that purpose, a set of compositions was prepared which were filled in a handheld spray device according to the invention (third embodiment). Further nitrogen was added to the reservoir. The ratio was 40% Nitrogen and 60 % composition, by volume. The set pressure at filling of nitrogen was 9.5 bar.

| **Ingredients composition (% by weight)** | **Ex.1** | **Ex.2** | **Ex.3** | **Ex.4** | **Ex.5** | **Ex.6** |
|---|---|---|---|---|---|---|
| Ethanol | 99 | 79 | 59 | 39 | 19 | 60 |
| Wasser | 0 | 20 | 40 | 60 | 80 | 35.09 |
| Parfüm | 1 | 1 | 1 | 1 | 1 | 1.4 |
| Glycerin | | | | | | 3 |
| Polyglyceryl-2 Caprylate | | | | | | 0.5 |
| Natural extract (Camellia Sinensis Leaf Extract) | | | | | | 0.01 |
| | | | | | | |
| | | | | | | |
| Dv(10) in µm | 22.8 | 25.2 | 32.2 | 28.6 | 22.5 | |
| Dv (50) in µm | 40.9 | 45.8 | 56.3 | 51.8 | 41.3 | 59.3 |
| Dv(90) in µm | 70.1 | 79.6 | 95.9 | 89.7 | 72.4 | |

The measurements were performed 48h after filling.

The following methodology was applied to analyze the particle size distribution of the obtained spray using fully filled spray handheld spray devices (according to the third embodiment of the invention).

The particle size distribution was measured by laser diffraction with a Malvern Panalytical Spraytec device. In laser diffraction, particle size distributions are calculated by comparing a sample's scattering pattern with an appropriate optical model using a mathematical inversion process. For these measurements the Mie Theory was used.

The droplet size of the spray was detected by measuring the light intensity scattered as a laser beam passes through a spray. The distance between the nozzle of the spray product and the laser beam was 15 cm. The distance between detector and the nozzle of the spray product was 18 cm. The vertical position of the nozzle of the spray device was adjusted such that the laser passes through the center of the spray jet.

The following settings were used:
Basic settings:
Measurement typ - Rapid
Data aquisation rate: 250 Hz
Lens type: 300 mm

### Optical properties:

Particle: Acetone - Refractive Index: 1,36 - Refractive Index (imaginary): 0 - Density: 0,80 Dispersant: Air - Refractive index: 1,00

### Data handling:

### Detector Range: 6 - 36

Scattering: (Threshold units) : 1
Measurement time: 2s

In total 6 measurements were performed per example formulation.

For volume weighted particle size distributions, such as those measured by laser diffraction, it is often convenient to report parameters based upon the maximum particle size for a given percentage volume of the sample. Percentiles are defined as XaB where:
X= parameter, usually D for diameter
a = distribution weighting, e.g. n for number, v for volume, i for intensity
B = percentage of sample below this particle size e.g. 50%, sometimes written as a decimal fraction i.e. 0.5

For example, the Dv50 would be the maximum particle diameter below which 50% of the sample volume exists - also known as the median particle size by volume.

The higher the value of the Dv10 or Dv50, ... the bigger the droplets.

Example: dv50 = 20 means 50% of the particle population is smaller than 20 µm. A value of 100 means that 50% of the particles are smaller than 100 µm. Since a normal distribution of the particles / droplets is assumed, a high value means a particle collective with larger diameters.

Thus, by analyzing the data provided in table above it can be noticed that the particle distribution shifts to larger particles for the examples Ex.2, Ex.3, Ex.4 and Ex.6. Ex.1 and Ex.5 are examples not according to the invention.

In a further consumer test two handheld spray devices according to the third embodiment were analyzed. The spray devices were prepared according to the description for the test above.

The devices S7 and D8 differ in the composition contained in the spray devices. S7 is a reference example while D8 is an example according to the invention. The ethanol/water ratio in S7 is 107:1. The ethanol/water ratio in D8 is 1.49:1. The total quantity of water and ethanol in the compositions exceeds 95% by weigh of all ingredients, based on the total weight of the compositions.

| **Ingredients composition (% by weight)** | **S7** | **D8** |
|---|---|---|
| Methyl Phenylbutanol | 0.8 | 0.15 |
| Butyloctanoic Acid | 1 | |
| Polyglyceryl-2 Caprate | | 0.5 |
| Ethylhexylglycerin | | 0.5 |
| Aqua + Propylene Glycol + Maris Limus Extract + Ostrea Shell Extract + Sodium Benzoate + Potassium Sorbate + Lactic Acid | 0.1 | |
| Persea Gratissima Oil | 0.1 | |
| Octyldodecanol | 1 | |
| Parfum | 1.5 | 1.3 |
| Ethanol | 95.02 | 57.72 |
| Aqua | 0.88 | 39.83 |

To evaluate the consumer feeling upon use of the handheld spray devices the formulars were tested by at least 140 consumers each. After four weeks of daily use the consumers were asked to evaluate the two products by answering the following questions. The values indicated in the table are the mean score.

| **Questions** | **S7** | **D8** |
|---|---|---|
| The product has a pleasant spray jet. (scale 1 to 7; 1= I disagree; 7= I agree) | 5.5 | 5.7 |
| The product leaves a pleasant skin feel. (scale 1 to 7; 1 = I disagree; 7= I agree) | 5.8 | 6.0 |
| The product feels cool on the skin. (scale 1 to 7; 1= I disagree; 7= I agree) | 5.7 | 5.9 |
| The product cares for my skin. (scale 1 to 7; 1= I disagree; 7= I agree) | 5.5 | 5.8 |

Accordingly it can be validly stated that the spray devices according to the invention allow for larger droplets to be formed in the spray, respectively the distribution of the droplets has been pushed to larger droplet sizes, while maintain a reasonable spray which leaves a pleasant skin feeling in the armpit.

Further compositions suitable to be used in the spray devices of the invention are presented in the following table:

| **Ingredients composition (% by weight)** | **Ex.7** | **Ex.8** | **Ex.9** | **Ex.10** | **Ex.11** | **Ex.12** |
|---|---|---|---|---|---|---|
| Ethanol | 75 | 52 | 58 | 44 | 59 | 60 |
| Wasser | 23 | 45 | 35 | 52 | 35 | 36 |
| Flüssiges Parfüm | 1.5 | 1 | 1.2 | 1.2 | 1.2 | 1.3 |
| Glycerin | 0.1 | 0.1 | 0.3 | 0.9 | 0.9 | 1.5 |
| 1,3-Propanediol | | | 5 | | | |
| Polyglyceryl-2 Caprylate | 0.3 | | | | 3.2 | 0.7 |
| Polyglyceryl-4 Caprate | | 1.5 | | 1.5 | | |
| Natural extract (Camellia Sinensis Leaf Extract) | | | | 0.1 | | |
| Ethylhexylglycerin | | | 0.4 | 0.3 | 0.5 | 0.5 |
| Persea Gratissima oil | | 0.1 | | | 0.2 | |
| epsilon-polylysine | 0.1 | | | | | |
| Polyquaternium-16 | | 0.3 | | | | |
| Octenidinhydrochloride | | | 0.1 | | | |

## Claims

1. Handheld spray device comprising
a) a body comprising a reservoir to house a total fill of materials;
b) an actuator comprising an actuator exit orifice;
c) a valve assembly in fluid communication with the actuator exit orifice and the reservoir;
d) a propellant stored in the reservoir, whereby the propellant has a concentration from 30% to 70% by volume of the total fill of materials stored within the reservoir and the propellant is nitrogen and/or compressed air; and
e) a composition, preferably a cosmetic composition, stored in the reservoir, whereby the composition comprises
a. ethanol, and
b. water,
wherein the ratio by weight between ethanol and water is in the range from 5:1 to 1:4.

2. Handheld spray device according to claim 1 **characterized in that** the valve assembly is configured such that a portion of the propellant from the reservoir is bled into the liquid composition being supplied to the actuator.

3. Handheld spray device according to claim 1 **characterized in that** valve assembly for an aerosol spray device, the assembly comprising:
a housing with internal walls defining a valve chamber, the chamber having a liquid inlet for fluid communication with liquid in the aerosol spray device, and a gas inlet for fluid communication with gas in the aerosol spray device; and a valve stem having proximal and distal ends, the proximal end received in the valve chamber and the distal end projecting through a sealed opening in the valve chamber, the valve stem including an outlet flow conduit with an outlet aperture at the distal end and, more proximally, at least one first stem inlet for liquid and at least one second stem inlet for gas;
wherein the housing includes a lip projecting inwardly from the internal walls to form a seal around a perimeter of the valve stem along at least a portion of the valve stem, wherein the valve chamber liquid inlet is proximal of the lip and
the valve chamber gas inlet is distal of the lip;
wherein the valve stem is moveable between:
a closed position in which there is no fluid communication between the valve chamber liquid inlet and the outlet conduit and there is no fluid communication between the valve chamber gas inlet and the outlet conduit; and
an open position in which the at least one first stem inlet is proximal of the lip so as to be in fluid communication with the valve chamber liquid inlet, and the at least one second stem inlet is proximal of the sealed opening in the valve chamber and at least partially distal of the lip so as to be in fluid communication with the valve chamber gas inlet, whereby a bubble laden flow is created in the flow conduit.

4. Handheld spray device according to claim 1 **characterized in that** valve assembly for an aerosol spray device, the assembly comprising:
a housing with internal walls defining a valve chamber, the chamber having a liquid inlet for fluid communication with liquid in the aerosol spray device, and a gas inlet for fluid communication with gas in the aerosol spray device; and a valve stem having proximal and distal ends, the proximal end received in the valve chamber and the distal end projecting through a sealed opening in the valve chamber, the valve stem including an outlet flow conduit with an outlet aperture at the distal end and, more proximally, at least one first stem inlet for liquid and at least one second stem inlet for gas;
wherein the housing includes a lip projecting inwardly from the internal walls to form a seal around a perimeter of the valve stem along at least a portion of the valve stem, wherein the valve chamber liquid inlet is proximal of the lip and the valve chamber gas inlet is distal of the lip;
wherein the valve stem is moveable between:
a closed position in which the at least one first stem inlet is distal of the lip and the at least one second stem inlet is distal of the sealed opening in the valve chamber, such that the at least one first stem inlet is not in fluid communication with the valve chamber liquid inlet and
such that the at least one second stem inlet is not in fluid communication with the valve chamber gas inlet; and
an open position in which the at least one first stem inlet is proximal of the lip so as to be in fluid communication with the valve chamber liquid inlet, and the at least one second stem inlet is proximal of the sealed opening in the valve chamber and at least partially distal of the lip so as to be in fluid communication with the valve chamber gas inlet, whereby a bubble laden flow is created in the flow conduit.

5. Handheld spray device according to one of the proceeding claims **characterized in that** the composition situated in the reservoir contains ethanol and water, wherein the ratio by weight between ethanol and water is in the range from 3:1 to 1:3 and preferably from 2:1 to 1:1.

6. Handheld spray device according to one of the proceeding claims **characterized in that** the total quantity of water in the composition is in the range from 18 to 62% by weight, more preferably from 25 to 55 % by weight and most preferably 30 to 50% by weight, calculated to the total weight of the composition.

7. Handheld spray device according to one of the proceeding claims **characterized in that** the total quantity of ethanol in the composition is in the range from 35 to 85% by weight, more preferably from 45 to 75% by weight and most preferably 50 to 65% by weight, calculated to the total weight of the composition.

8. Handheld spray device according to one of the proceeding claims **characterized in that** the composition comprises one further liquid fragrance material.

9. Handheld spray device according to one of the proceeding claims **characterized in that** the composition contains from 0.2 to 4% by weight, more preferably from 0.3 to 3% by weight and most preferably of 0.4 to 2.5% by weight of at least one liquid fragrance material, calculated to the total weight of the composition.

10. Handheld spray device according to one of the proceeding claims **characterized in that** one or more additional deodorizing active ingredient is contained in the composition.

11. Handheld spray device according to claim 10 **characterized in that** the deodorizing active ingredients is be selected from cationic polymers, in particular polyquaternium-16, polyaminopropyl biguanide and epsilon-polylysine.

12. Handheld spray device according to claim 10 **characterized in that** deodorizing active ingredients is selected from the group consisting of octenidinhydro chloride, alexidindihydro chloride, benzyl alcohol, benzalkonium chloride, cetyltrimethylammonium chloride, silver citrate, triclosan, ethylhexylglycerin, triethyl citrate, 2-butyloctanoic acid, Methylphenylbutanol, phenoxyethanol and zinc ricinoleate.

13. Handheld spray device according to any of the proceeding claims **characterized in that** the pressure in the reservoir is in the range from 12 bar to 3 bar, preferably 10 bar to 4 bar and most preferred from 9 to 6 bar for the case the spray device is fully filled and not used yet.

14. Handheld spray device according to any of the proceeding claims **characterized in that** the composition comprises at least one polyol having 3 to 10 carbon atoms, preferably selected from the group consisting of propanediol, propylene glycol, butylene glycol, glycerol,1,2 propanediol, 1,2 hexanediol, 1,2 heptanediol and decylene glycol.

15. Handheld spray device according to claim 14 **characterized in that** at least one polyol having 3 to 10 carbon atoms is contained in a total quantity in the range from 0.1 to 6% by weight, more preferably 1 to 5% by weight and most preferably 2 to 4% by weight, calculated to the total weight of the composition.

16. Use of the handheld spray device according to any of the proceeding claims to spray the cosmetic composition to the human body, whereby the spray is released by actuating the actuator.
